# EUROPEAN PATENT SPECIFICATION

(11) **EP 1 430 138 B1**
(45) Date of publication and mention of the grant of the patent: **18.01.2006**
(21) Application number: 01976603.9
(22) Date of filing: 27.09.2001
(51) Int. Cl.: C12P 7/62, A61K 31/22, C12R 1/465

(54) **PROCESS FOR PRODUCING PRAVASTATIN SODIUM SALT USING STREPTOMYCES FLAVIDOVIRENS DSM 14455**
VERFAHREN ZUR HERSTELLUNG VON PRAVASTATIN-NATRIUMSALZ MITTELS STREPTOMYCES FLAVIDOVIRENS DSM 14455
PROCEDE DE PRODUCTION D'UN SEL DE SODIUM DE PRAVASTATINE AU MOYEN DE STREPTOMYCES FLAVIDOVIRENS DSM 14455

(43) Date of publication of application: 23.06.2004
(73) Proprietor: Biocon Limited, Bangalore 561 229 (IN)
(72) Inventor: GURURAJA, Ramavana, Bangalore 561 229, Karnataka (IN); GOEL, Anuj, Bangalore 561 229, Karnataka (IN); SRIDHARAN, Madhavan, B angalore 561 229, Karnataka (IN); MELARKODE, Ramakrishnan, Bangalore 561 229, Karnataka (IN); KULKARNI, Madhav, Bangalore 561 229, Karnataka (IN); POORNAPRAJNA, Acharya, Bangalore 561 229, Karnataka (IN); SATHYANATHAN, Deepthy, Bangalore 561 229, Karnataka (IN); GANESH, Sambasivam, Bangalore 561 229, Karnataka (IN); SURYANARAYAN, Shrikumar, Bangalore 561 229, Karnataka (IN)
(74) Representative: Freyria Fava, Cristina
(86) International application number: PCT/IN2001/000161
(87) International publication number: WO 2003/027302

(56) References cited:
- EP-A- 0 317 292
- WO-A-99/10499
- US-A- 4 346 227
- HOSOBUCHI M ET AL: "APPLICATION OF COMPUTER TO MONITORING AND CONTROL OF FERMENTATION PROCESS: MICROBIAL CONVERSION OF ML-236B NA TO PRAVASTATIN" BIOTECHNOLOGY AND BIOENGINEERING, INTERSCIENCE PUBLISHERS, LONDON, GB, vol. 42, no. 7, 1993, pages 815-820, XP002036625 ISSN: 0006-3592
- WATANABE I ET AL: "Molecular approaches for production of pravastatin, a HMG-CoA reductase inhibitor: transcriptional regulation of the cytochrome P450sca gene from Streptomyces carbophilus by ML-236B sodium salt and phenobarbital" GENE: AN INTERNATIONAL JOURNAL ON GENES AND GENOMES, ELSEVIER SCIENCE PUBLISHERS, BARKING, GB, vol. 210, no. 1, 27 March 1998 (1998-03-27), pages 109-116, XP004117457 ISSN: 0378-1119
- HAYAKAWA Y ET AL: "A NEW DEPSIPEPTIDE ANTIBIOTIC, CITROPEPTIN" AGRICULTURAL AND BIOLOGICAL CHEMISTRY, JAPAN SOC. FOR BIOSCIENCE, BIOTECHNOLOGY AND AGROCHEM. TOKYO, JP, vol. 54, no. 4, 1990, pages 1007-1011, XP002913371 ISSN: 0002-1369

## Description

### FILED OF THE INVENTION:

The present invention relates to a process for the manufacture and purification of Pravastatin sodium salt using a new microorganism *Streptomyces flavidovirens* DSM 14455.

### BACKGROUND OF THE INVENTION

Lovastatin, pravastatin and compactin and derivatives and analogs thereof are known to be potent HMG-CoA reductase inhibitors and are used as antihyper-cholesterolemic agents. Lovastatin, compactin and pravastatin are produced by fermentation using microorganisms of different species belonging to *Aspergillus, Penicillium* and *Streptomyces* genera respectively.

The purity of the active ingredient is an important factor for manufacturing the safe and effective pharmaceutical, especially if the pharmaceutical product must be taken on a long-term basis in the treatment or prevention of high plasma cholesterol. The accumulation of the impurities from the pharmaceuticals of lower purity may cause many side effects during the medical treatment.

Among all the statins produced by microorganisms, pravastatin is the drug of choice as it has stronger and highly tissue selective inhibition of cholesterol synthesis (Tsujita *et. al,* Biochim Biophy Acta, 1986, 877, 50-60). Pravastatin is produced by microbial hydroxylation of its precursor, Compactin (also called ML-236B). This bioconversion is carried out by a number of microorganisms e.g. Streptomyces (US 5,179,013, US 4,448,979), *Nocardia, Amycolata, Saccharopolyspora, Amycolatopsis, Saccharothrix, Gilbertella* (EP 0649907, WO 99/60151), *Actinomadura* (WO 96/40863), *Mortierella* (WO 00/46175), *Nocardia* (US 5,830,695) and *Bacillus* sp. (US 6,245,535, WO 99/07827). A number of species of Streptomyces, e.g. *S. carbophilus, S. hastedii* (JP 4,349,034), *S. flavovirens* (WO 99/10419), *S. rosenchromogenous* (US 4,346,227), *S. californicus* (EP 649907) and *S*. *exfoliatus* (WO 98/45410) are known to carry out this bioconversion.

The bioconversion is cytochrome p450 dependent system and the enzymes are induced by presence of compactin in the medium (Matsuoka et al, European Journal of Biochemistry, 1989: 184: 707-713; Serizawa *et. al*, In: Biotechnology of antibiotics; WR Strohl (editor) 1997). Compactin has to be often added into the seed medium for efficient bioconversion (WO 98/45410). Attempts have been made to clone and express this system by recombinant DNA techniques in a fungal host (e.g. *Penicillium citrinum*) for one step *de novo* production of pravastatin (WO 99/10419). However, the yields are not economically viable. Bioconversion using *Streptomyces* species is still the most efficient method of pravastatin production currently.

### Disclosure of the invention

The object of the present invention is to provide a process for efficient conversion and purification of compactin to pravastatin sodium using a new strain of Streptomyces.

Accordingly the present invention provides a process for the manufacture and purification of Pravastatin sodium salt, characterized by
(i) preparing seed inoculum of a strain of *Streptomyces flavidovirens* able to 6β-hydroxylate a compound of formula II,
(ii) transferring the said seed inoculum to a production medium,
(iii) subjecting the said production medium to fermentation,
(iv) feeding the substrate to be transformed in the said production medium at different intervals,
(v) controlling the pH during fermentation by feeding carbon sources,
(vi) fermenting the substrate until the end of bioconversion,
(vii) extracting the whole cell broth and separating the compound of formula I, and
(viii) isolating the compound of formula I.

The process wherein the strain of *Streptomyces flavidovirens* is *Streptomyces flavidovirens* strain deposited at DSMZ (Deutsche Sammlung von Mikro-organismen und Zellkulturen GmbH) under accession number QSM: 14455.

The process wherein the said inoculum used for the seed is a spore suspension or a vegetative mycelium.

The process wherein the constituents of the said seed medium is selected from malt extract and peptone.

The process wherein the pH of said seed medium is 6.0 to 7.5 before sterilization.

The process wherein the seed medium is incubated at 25 to 35 deg C for 40 to 55 hours.

The process wherein the constituents of said production medium is selected from dextrose monohydrate, peptone and yeast extract.

The process wherein the said production medium has a pH of 6.0 to 7.5 before sterilization.

The process wherein the said production medium is incubated at 24 to 35 deg C. for 48 to 148 hours.

The process wherein the substrate used for feeding is compactin, a salt of compactin or a derivative of compactin.

The process wherein the pH is controlled by feeding a carbon source selected from a saccharide or glycerol.

The present invention has the following advantages over the other reported methods:
(i) New strain of *Streptomyces flavidovirens* DSM 14455.
(ii) Higher bioconversion rate making the process economically attractive.
(iii) Fewer steps for the isolation and purification to get the pure product.

Streptomyces has been the most commonly used microorganism as its cytochrome system has been well studied (EP 281245, US5,830,695). Among the Streptomyces, *S*. *flavidovirens* offers unique advantage for bioconversion. Unlike *S*. *exfoilafus* (WO 98/45410) it does not need induction during vegetative phase. Here the bioconversion is between 40 and 90 % but more..... often between 60 and 80 % at the compactin concentrations in the vegetative broth between 0.05 and 10 g/L but more often between 3 and 6 g/L.

Unlike many cultures reported in prior art (e.g. *Mortierella maculata,* WO 00/46175) where the bioconversion is slow and is carried out over 12 days of fermentation, *S*. *flavidovirens* carries out conversion within 24 hrs to 7 days but preferably between 2 to 5 days.

The invention will now be described with reference to the following examples:

### Example 1

### Seed inoculum preparation:

About 100 µL spore suspension of *Streptomyces flavidovirens* DSM 14455 made by adding 3 mL of sterile water to a culture slant, is added to a 250 mL Erlenmeyer flask containing 35 mL of medium containing (in g/L) Malt extract 30 and peptone 5. pH is adjusted to 6.8 before sterilization. The seed flasks are incubated at 28 deg C on a rotary shaker (200-rpm) for 48 hours.

### Example 2

### Seed inoculum preparation:

Seed inoculum was prepared in the same way as in example 1 but the spore suspension was replaced by 1 mL of vegetative mycelium stored in glycerol.

### Example 3

### Bioconversion to Pravastatin:

In a 250 mL Erlenmeyer flask, about 0.5 mL of seed inoculum from example 2 was transferred to 35 mL of production medium containing (in g/L) Dextrose monohydrate 20, Peptone 10 and yeast extract 1. Before inoculation, the pH of the medium was adjusted to 7.0 and the flasks were sterilized for 30 min at 121 deg C.

The flasks were then incubated on a rotary shaker (200-rpm) at 28 deg C. After 2 days of incubation, sterile sodium salt of compactin solution was added along with sterile dextrose feed (50% w/v). The bioconversion was estimated after 24 hrs by harvesting one of the multiple flasks running under similar conditions. This procedure was repeated every 24 hrs till 3 mg/mL of compactin was fed cumulatively. Maximum amount of pravastatin accumulated at the end of experiment was about 1.5mg/mL.

### Example 4

### Bioconversion to Pravastatin:

The bioconversion was carried out in a 250 mL Erlenmeyer flask in the same way as described in example 3 but 7 mg/mL of dextrose feed was added every 24 hours along with compactin solution. About 2.0 mg/mL of pravastatin was detected in the flasks after 120 hrs of incubation.

### Example 5

### Bioconversion to Pravastatin:

The bioconversion was carried out in a 2L-stirred tank bioreactor with 1.7 L medium. In addition to the medium components described in example 3, 0.1% (v/v) of silicone antifoam was added before autoclaving the medium.

Seed inoculum (3.5%) was transferred aseptically to the bioreactor and the culture was allowed to grow at 28 deg C. The dissolved oxygen concentration was maintained above 25% of saturation. After 48 hrs of incubation, about 1 mg/ml of compactin feed was added every day. Along with every compactin feed, dextrose feed was also added. The pH of the reaction mixture was maintained between 7.6 to 8.0 by addition of dextrose feed on demand. At the end of 4 days, 1.6 g/L of pravastatin was produced.

### Example 6

### Bioconversion to Pravastatin:

The bioconversion was carried out in the similar way as in example 5 but no sugar was added along with compactin feed.

About 0.5 mg/ml of compactin feed was added in every shot addition. The pH of the reaction mixture was maintained between 8.4 and 8.6 by addition of dextrose feed on demand. About 46% of the 2.9 g/L of compactin fed to fermentor was detected as pravastatin.

### Example 7

### Bioconversion to Pravastatin:

The bioconversion was carried out as described in example 3 but the compactin was added as ammonium salt. 0.9 g of the total compactin fed (2.9 g/L) was assayed as pravastatin.

### Example 8

### Extraction of broth:

The whole cell broth after fermentation was obtained and the pH was adjusted to 12 and held for 1 hr. 90% of the total product present was extracted into supernatant.

### Example 9

### Scale up studies on hydrophobic interaction resin:

A hydrophobic interaction resin - SP825 (MITSUBISHI CHEMICAL CORPORATION) was packed in a XK 26/70 (Pharmacia) column. The pH 12 extracted and filtered broth from example 8 was passed through a pre-equilibrated column and pravastatin was bound. The washing step was done using pH 12 water. The elution was done with methanol. The overall recovery from the broth extract was 92%.

### Example 10

### Separation of pravastatin using secondary amine:

80 ml of methanolic extract from the HIC (hydrophobic interaction chromatography) resin containing 10g pravastatin sodium salt was acidified to pH 4 using dilute HCl and the pravastatin in the acid form was extracted into equal volume of ethyl acetate.

To this, 120 mole % of a dibenzylamine was added, stirred for half-hour and then chilled at 4 deg C for 1-2 hours. The dibenzyamine salt of pravastatin was separated by filtration. The crystals were washed with ethyl acetate, filtered and dried. Dibenzylamine salt of pravastatin containing 9g equivalent of pravastatin acid was obtained.

### Example 11

### Precipitation of sodium salt of pravastatin using Sodium caprylate:

Around 5g of amine salt of pravastatin was dried and dissolved in 20 ml of 10 % NaOH solution and washed with ethyl acetate and pH was adjusted to 4 using dilute hydrochloric acid. This was further extracted into equal volume of ethyl acetate.

The ethyl acetate layer was washed with brine, activated carbon was added and stirred for 30 min at room temperature. It was filtered and washed with ethyl acetate. The ethyl acetate solution was dried over anhydrous sodium sulphate.

To the ethyl acetate solution 1.2 gms of sodium caprylate was added and stirred for 2 hrs at room temperature. Then 12.5 ml of acetonitrile was added and stirred for 1hr. The reaction mixture was cooled to 5 deg C, and stirred for 1hr.

The precipitate of the sodium salt of pravastatin were filtered and washed with chilled acetonitrile. The compound was dried under vacuum to yield pravastatin sodium with a yield of 90% and an assay purity of greater than 99%.

### Example 12

### Purification of Pravastatin:

The broth (10000L) was acidified to pH = 4 by adding 50% ortho phosphoric acid and equal volume of ethyl acetate was added. The layers were separated and the organic layer was washed with water and concentrated under reduced pressure to give a total volume of about 300L and was stirred under reflux for 24h. Cool to room temperature and washed with 5% sodium bicarbonate solution, water and concentrated under reduced pressure to 125L. The residue was chilled to 0°C and stirred for 2h. The solid obtained was filtered to give the lactone.

To 1 Kg lactone obtained from the above step, methanol (1L) and 10% sodium hydroxide (2L) were added and stirred for 0.5h at room temperature. Water (1L) and ethyl acetate (2L) were added and the contents were stirred for 10min at room temperature. Ethyl acetate layer was separated and discarded. The pH of the aqueous layer was carefully adjusted to 4 using 6N HCl and was extracted with ethyl acetate (4L). The layers were separated, the ethyl acetate layer washed with brine, activated charcoal was added, filtered and dried over sodium sulfate. To the filtrate sodium caprylate (347g) was added and the contents were stirred for 1h and acetonitrile (2.5L) was added and stirring was continued for additional 2h, chilled to 0°C, and the solid precipitate of the sodium salt of pravastatin was filtered and dried at 40°C under vacuum.

The crude sodium salt was dissolved in 2L of water and acetonitrile was added (30L) over a period of 2h. The contents were chilled to 0°C and were stirred for 4h at 0°C, filtered and the solid was washed with acetonitrile. The solid was dried under vacuum at 40°C to give pharmaceutical grade pravastatin sodium salt.

### Example 13

### Purification of Pravastatin:

To the ethyl acetate layer as obtained in example 12, instead of sodium caprylate, sodium acetate was addedand processed to give pharmaceutical grade pravastatin sodium salt.

### Example 14

### Purification of Pravastatin:

100 gm crude pravastatin lactone, obtained in a similar way as in example 12 was added to 300 ml alkaline methanolic solution. pH of the solution was adjusted to 4 and the pravastatin acid was extracted into 400 ml Ethyl acetate. To this ethyl acetate solution 120 mole % dibenzylamine was added, stirred for 1 hour and chilled at 4 deg C and filtered. The crystals were dissolved in methanol, pH was adjusted to 4 by dilute hydrochloric acid and pravastatin was extracted into ethyl acetate. To this 90 mole% sodium caprylate was added and stirred well. Crystals of the sodium salt precipitated out when acetonitrile was added to the solution. This was chilled for 1 hour. The product recovered was 70% with greater than 99 % assay purity.

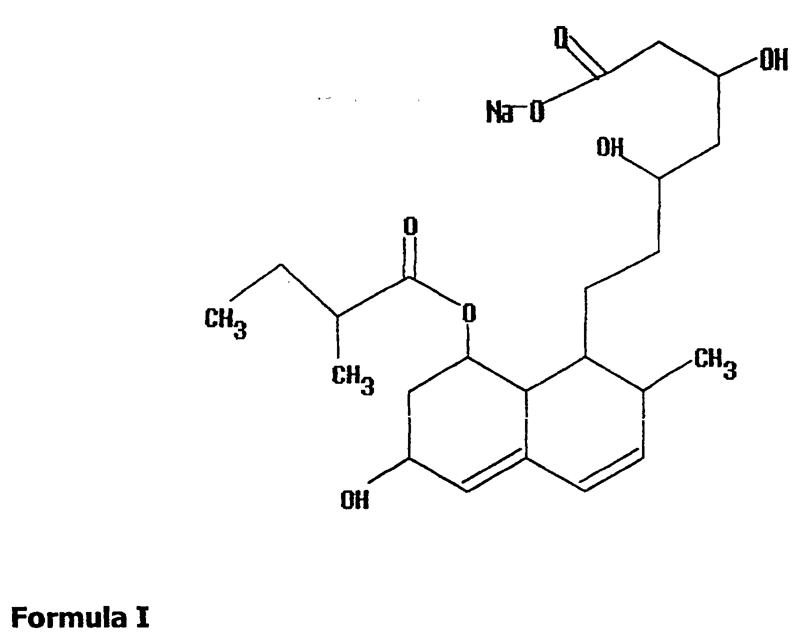

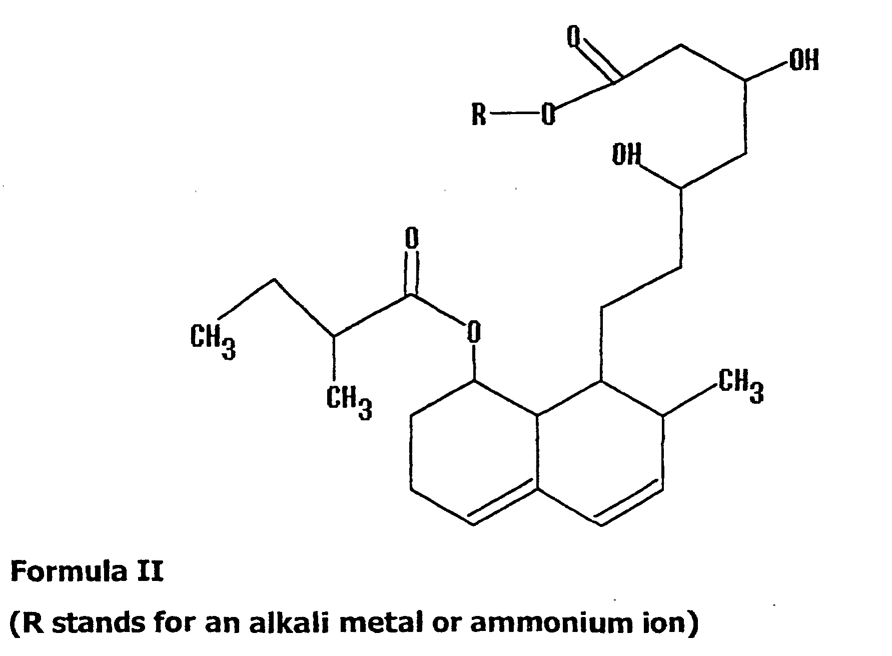

## Claims

1. A strain *Streptomyces flavidovirens* DSM 14455.

2. A microbial process for the preparation of the compound of formula I from a substrate compound of general formula II, wherein R represents an alkali metal or ammonium ion, comprising steps of
(i) preparing seed inoculum of a strain of *Streptomyces flavidovirens* able to 6β-hydroxylate a compound of formula II, 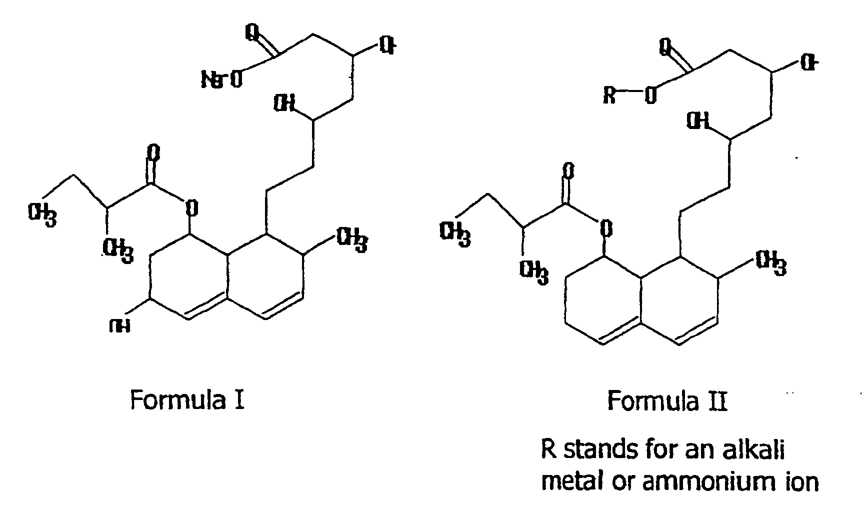
(ii) transferring the said seed inoculum to a production medium,
(iii) subjecting the said production medium to fermentation,
(iv) feeding the substrate to be transformed in the said production medium at different intervals,
(v) controlling the pH during fermentation by feeding carbon sources,
(vi) fermenting the substrate until the end of bioconversion,
(vii) extracting the whole cell broth and separating the compound of formula I, and
(viii) isolating the compound of formula I.

3. The process of claim 2, wherein the strain of *Streptomyces flavidovirens* is *Streptomyces flavidovirens* strain deposited at DSMZ (Deutsche Sammlung von Mikro-organismen und Zelikulturen GmbH) under accession number DSM 14455.

4. The process of claim 2 wherein the said inoculum used for the seed is a spore suspension or a vegetative mycelium.

5. The process of claim 2 wherein the substrate used for feeding is compactin, a salt of compactin or a derivative of compactin.

## Revendications

1. Souche de *Streptomyces flavidovirens* DSM 14455.

2. Processus microbien pour la préparation du composé de formule I à partir d'un composé substrat de formule générale II, dans laquelle R représente un métal alcalin ou un ion ammonium, comprenant les étapes de
(i) préparation d'un inoculum d'ensemencement d'une souche de *Streptomyces flavidovirens* capable de 6β-hydroxyler un composé de formule II, 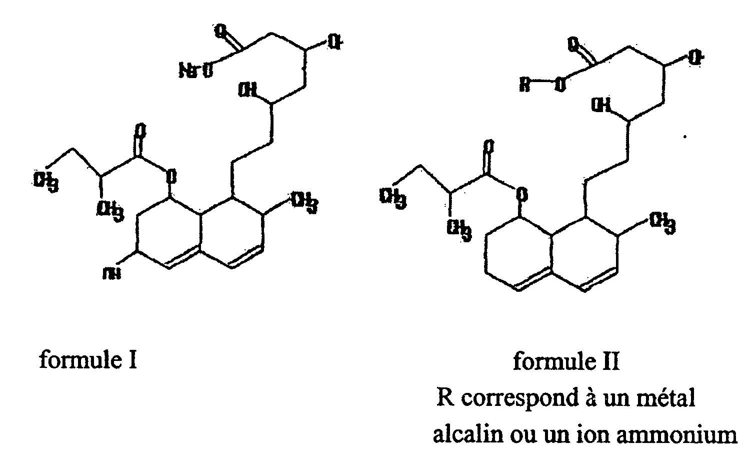
(ii) transfert dudit inoculum d'ensemencement à un milieu de production,
(iii) soumission dudit milieu de production à une fermentation,
(iv) alimentation du substrat à transformer dans ledit milieu de production à différents intervalles,
(v) contrôle du pH durant la fermentation en introduisant des sources de carbone,
(vi) fermentation du substrat jusqu'à la fin de la bioconversion,
(vii) extraction du bouillon de cellules entières et séparation du composé de formule I, et
(viii) isolement du composé de formule I.

3. Processus selon la revendication 2, dans lequel la souche de *Streptomyces flavidovirens* est la souche de *Streptomyces flavidovirens* déposée au DSMZ (Deutsche Sammlung von Mikro-organismen und Zellkulturen GmbH) sous le numéro d'accès DSM 14455.

4. Processus selon la revendication 2, dans lequel ledit inoculum utilisé pour l'ensemencement est une suspension de spores ou un mycélium végétatif.

5. Processus selon la revendication 2, dans lequel le substrat utilisé pour l'alimentation est la compactine, un sel de compactine ou un dérivé de la compactine.

## Patentansprüche

1. Einen Stamm *Streptomyces flavidovirens* DSM 14455.

2. Einen mikrobiologischen Prozess für die Zubereitung der Verbindung der Formel I aus einer Substratverbindung der allgemeinen Formel II, worin R für ein Alkalimetall oder Ammoniumion steht, umfassend die folgenden Schritte:
(i) Zubereiten eines Impfinokulums eines Stammes von *Streptomyces flavidovirens,* der in der Lage ist, eine Verbindung der Formel II an der Position 6β zu hydroxylieren, 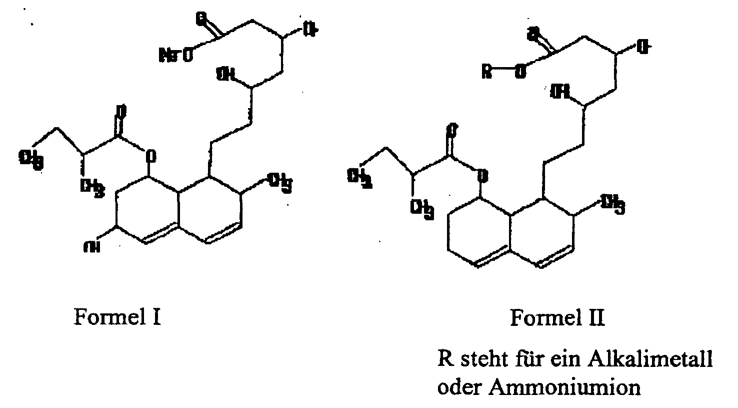
(ii) Übertragen dieses Impfinokulums in ein Produktionsmedium,
(iii) Aussetzen dieses Produktionsmediums an Fermentation,
(iv) Einspeisen des zu transformierenden Substrates in dieses Produktionsmedium in unterschiedlichen Intervallen,
(v) Kontrollieren des pH-Wertes während der Fermentation durch das Einspeisen von Kohlenstoffquellen,
(vi) Fermentieren des Substrates bis zu dem Ende der Bioumwandlung,
(vii) Extrahieren der Gesamtzellnährlösung und Abscheiden der Verbindung der Formel I, und
(viii) Isolieren der Verbindung der Formel I.

3. Prozess nach Anspruch 2, worin der Stamm von *Streptomyces flavidovirens* der bei der DSMZ (Deutsche Sammlung von Mikroorganismen und Zellkulturen GmbH) unter der Zugangsnummer DSM 14455 hinterlegte *Streptomyces-flavidovirens-*Stamm ist.

4. Prozess nach Anspruch 2, worin dieses für das Impfen verwendete Inokulum eine Sporensuspension oder ein vegetatives Myzel ist.

5. Prozess nach Anspruch 2, worin das für das Einspeisen verwendete Substrat Compactin, ein Compactinsalz oder ein Compactinderivat ist.
